# EUROPEAN PATENT SPECIFICATION

(11) **EP 2 229 196 B1**
(45) Date of publication and mention of the grant of the patent: **20.02.2013**
(21) Application number: 08865639.2
(22) Date of filing: 19.12.2008
(51) Int. Cl.: A61M 25/10, A61M 3/02

(54) **PRESSURE-CONTROLLED BALLOON CATHETER**
DRUCKGESTEUERTER BALLONKATHETER
CATHÉTER À BALLONNET COMMANDÉ PAR PRESSION

(30) Priority: 21.12.2007 DK 200701849
(43) Date of publication of application: 22.09.2010
(73) Proprietor: Coloplast A/S, 3050 Humlebæk (DK)
(72) Inventor: TANGHOEJ, Allan, DK-2980 Kokkedal (DK)
(86) International application number: PCT/DK2008/050331
(87) International publication number: WO 2009/080051

(56) References cited:
- US-A- 3 065 750
- US-A- 5 074 842
- US-A1- 2005 288 684

## Description

The invention relates to a pressure controlled balloon catheter or probe. The probe is to be used in connection with anal irrigation. The invention also relates to a method for performing anal irrigation.

### Background

Anal irrigation is one of a number of treatments used to aid people with bowel problems. People suffering from bowel problems are often paralyzed, typically due to spinal cord injuries, and confined to a wheelchair or hospitalized. In these situations, often the peristaltic functions, i.e. the reflexes and muscles of the bowel, cannot be stimulated correctly. This results in constipation or random discharge of bowel contents. By using anal irrigation a stimulation of the peristaltic movements of the colon can be provided.

To perform such anal irrigation, a device comprising an anal probe, also called anal catheter, rectal catheter or speculum, is provided. The anal probe is inserted into the rectum through the anus. The anal probe is typically retained in the rectum by retention means, most commonly a balloon, which is inflated against the wall of the rectum. A liquid, such as water or a saline solution, is then introduced into the rectum through the anal probe. The amount of liquid is generally up to 1.5 liters, depending on the person.

The anal probe is then removed followed by a discharge from the colon through the rectum. In some cases during anal irrigation the expansion of the rectum and the sphincter or the early stimulation of the peristaltic movements of the rectum may result in that the inflated balloon, or other fixating means, does not provide proper sealing and discharge of the rectum will thus occur around the anal probe. This undesired discharge can be very unpleasant for the patient as well as for the caring personnel performing the anal irrigation.

### Description of related art

US20050288684 shows an intra-bronchial device having a pressure relief system that minimizes the risk of injuries. Intra-bronchial device has a pressure relief valve that opens when the differential pressure between the collapsing lung portion on the distal side of the device and the lung portions on the proximal side of the device exceeds a desired amount.

US3065750 shows a sanitary douche which may be dilated upon passage therethrough of the fluid employed therewith. More specifically, it deals with a cylindrical unit surrounded by plastic bags or balloons which become dilated upon passage therethrough of sanitizing liquid under pressure.

US5074842 provides a system for anal irrigation which comprises a tube and a balloon for retaining the tube in the rectum. The balloon is a specially shaped balloon designed to retain the tube in the rectum and it can be inflated after the tube is inserted. Air to the balloon is delivered through a syringe valve into a line which communicates with an air channel which allows the colon sweeping solution to enter the patient, and a second channel, which delivers or removes air from the balloon used to retain the tube in place

### Summary of the Invention

The invention concerns a probe for use in connection with anal irrigation, where the probe has two distinct parts - a tip part and a shaft part, which may be separate from each other so that the tip part can move longitudinally with respect to the shaft part. The two parts may also be one integral element. Each of the parts has a water channel and in a first configuration of the probe, the water channels are not in contact with each other while in a second configuration of the probe the water channels are in contact with each other. The probe is provided with a pressure-sensitive valve controlling when the water channels are in contact with each other. The probe further comprises retention means for retaining the probe in the rectum or urethra.

### Detailed Description of the Invention

In a first aspect the invention relates to an anal probe for performing anal irrigation having a tip part and a shaft part, which probe comprises
- a tip part having a tip channel with an inlet and an outlet
- a shaft part having a shaft channel with an inlet, an outlet and a connector adapted for connection to fluid supply means
- retention means for retaining the probe in the rectum, which retention means is attached to the tip part and the shaft part and a lumen is defined by the retention means, the tip part and the shaft part,
   where the outlet of the shaft is in fluid communication with the lumen of the retention means and where the probe comprises a pressure-sensitive valve adapted for providing the probe with
   - a first configuration in which the inlet to the tip channel is not in fluid communication with the lumen, and
   - a second configuration in which the inlet to the tip channel is in fluid communication with the lumen.

Such a probe is particularly easy to use, as a user only needs to attach a fluid supply including a pump to the probe and pump the irrigation liquid into the probe. Then the retention means will expand until the pressure in the retention means has reached a predetermined level. At this point, the pressure-sensitive valve will move the probe from the first configuration into the second configuration thereby providing communication between the lumen of the retention means and the tip channel. In this second configuration irrigation liquid will be able to flow through the tip channel and into the rectum or stoma.

When such a probe is to be used, only one pumping action is needed as the same action will both activate the retention means and pump irrigation fluid into the rectum.

Furthermore, such a pressure-sensitive valve ensures that the balloon cannot be inflated to an excessive degree as the valve will control the pressure inside the balloon. Thus in such a probe the balloon will not be likely to burst.

The longitudinal part of the probe may be in the form of a rectal catheter having a coating so as to make the insertion easier. Typically the catheter will be a cylindrical body having a size of about 8-16 mm as the diameter such as 10 mm and a length of about 70-200 mm such as about 150 mm.

The tip part comprises a generally cylindrical part having a rounded tip with eyelets in the proximal end, where the proximal end is defined as the insertion end. The tip part has a through-going water channel (a tip channel) having an inlet in or near the distal end and an outlet in the proximal end. The eyelets communicate with the water channel of the tip part just as an outlet of the water channel corresponds to an eyelet of the tip. The tip part may be provided with a cap situated at the outmost tip portion. The cap would to a certain degree cover the eyelets during insertion, thereby preventing the eyelets from being blocked by feces present in the rectum or colon (for anal and stomal irrigation respectively). Furthermore, the tip may be provided with several eyelets such as up to 10 or 15 or even more - thereby reducing the risk that all of them are blocked at the same time.

The shaft part includes a generally cylindrical part having a connector in the distal end for connecting the shaft part to the fluid supply. The shaft part also includes a water channel (a shaft channel) having an inlet in connection with the connector and an outlet near the proximal end.

In an embodiment of the invention, the tip part and the shaft part are separate parts.

In another embodiment of the invention, the tip part and the shaft part are one integral part.

As mentioned earlier, the probe comprises two water channels, a tip channel and a shaft channel. For a lumen defined by the retention means, the tip part and the shaft part, the inlet of the tip channel will not be in communication with this lumen in a first configuration of the probe and in communicating with this lumen in a second configuration. In other words, in a first configuration of the probe, the inlet of the tip channel is closed and in a second configuration of the probe, the inlet of the tip channel is opened. The transition between the two configurations is controlled by the pressure-sensitive valve.

When the tip part and shaft part are separate elements, they may be joined through a protrusion in one part and an indentation in the other part. This connection may be established only in either the first or the second position.

In an embodiment the tip part and the shaft part are elongated elements defining a longitudinal direction and the tip part is displaced longitudinally with respect to the shaft part in the second configuration of the probe. By longitudinally displacement is meant a displacement generally in the longitudinal direction. This first configuration may correspond to a contracted position and the second configuration may correspond to an extended position. This means that the tip part and the shaft part have a distance between them in the extended position and are in contact with each other in the contracted position. The shaft channel has an outlet in communication with the aforementioned lumen. In a first configuration of the probe, there is no communication between the two water channels of the probe, as the inlet to the tip channel is out of communication with the lumen. In the second configuration of the probe, the irrigation fluid may access the inlet of the tip channel and hence reach the outlet of this channel (in connection with the eyelets) and flow further into the rectum.

One way of doing this is that the tip part and the shaft part will be pulled from each other by applying a pressure to the tip part and thus pushing the tip part from the shaft part. When the tip part has separated a certain distance from the shaft part, access to the tip channel will be provided and water will flow through the tip channel and further into the rectum. Transverse and longitudinal directions are in this context defined as the directions of the probe so that the longitudinal direction corresponds to the lengthwise direction of the probe and the transverse direction is transverse thereto. The probe is a generally cylindrical element having an inlet in one end and an outlet in the other, both generally along the axis of the cylindrical element. This axis defines the lengthwise direction.

Another way of doing this is that the pressure-sensitive valve includes a blocking element which in the first configuration of the probe covers the inlet to the tip channel and in the second configuration of the probe is displaced with respect to the inlet. Thereby the tip part and the shaft part may be made as one integral element. This construction may be advantageous if the rectum is blocked by a blockage of faeces. In this situation, when the probe is inserted, it may be difficult for the tip to move forward due to the blockage. Thus a probe, where the tip part is not movable and where the tip part includes other means for opening for communication for the irrigation liquid through the tip-channel, may be easier to use.

If the tip part and shaft part are separate parts, they may define contact surfaces in contact with each other in the first configuration of the probe. These contact surfaces may be provided as polished surfaces so that they define close and intimate contact to provide for a sealing between the surfaces. Such a sealing between smooth surfaces may be sufficient to ensure that in this first configuration most of the irrigation fluid is prevented from flowing through the tip part. In a related embodiment the contact surfaces may be provided with an additional sealing element in form of a gasket.

In an embodiment the inlet to the tip part is placed in the contact surface of the tip part. This provides for a particularly simple solution as the tip part may then be formed as a simple cylindrical element having a rounded tip. In another embodiment either the tip part or the shaft part has a protrusion and the other part has an indentation corresponding to the protrusion. This provides a solution where the inlet to the water channel of the tip part is hidden in either the indentation or at the protrusion depending on whether the tip part has an indentation or a protrusion. Such solutions increase the ability of the probe to keep the inlet to the tip part closed in the first configuration. If the tip part has a protrusion and the shaft part has a corresponding indentation, then the inlet may be provided in the sidewall of the protrusion. In the first configuration, this inlet is hidden and closed due to the contact surfaces and the contact between the sidewalls of the protrusion and the sidewalls of the indentation in the shaft part. If the tip part has an indentation and the shaft part has a corresponding protrusion the inlet may be provided anywhere in the indentation and it may be accessed or opened when the protrusion is completely removed from the indentation. In a related embodiment the protrusion of the shaft portion may be provided with an additional water channel having an inlet in a sidewall of the protrusion and an outlet in the end of the protrusion. In this embodiment the protrusion need not be completely removed from the indentation in order to create access to the inlet of the tip part.

In an embodiment the protrusion comprises an abutment and the indentation comprises a cavity corresponding to the abutment so that the abutment prevents the protrusion from completely being removed from the indentation.

The outlet from the shaft water channel communicates with an interior of the retention means, e.g. a lumen of a balloon.

The probe is provided with retention means, which may be in the form of a balloon-element. The balloon-element may be made of PU (Poly-Urethane), latex or PVC (Polyvinyl-chloride) although other materials having similar properties may be used. Other types of material may be silicone elastomers (e.g. LSR, HTV, RTV, Addition Cure or Condensation Cure), natural rubber latex, isoprene rubber, chloroprene rubber (e.g. Neoprene ®), PU (poly-urethane, e.g. TPU or cross-linked), styrenic elastomers (TPE-S, e.g. Kraton ®, Septon ®, Sibstar ® reactor materials or compounds) and poly-ether block amide (TPE-A), e.g. Pebax ®).

In an embodiment the material of the balloon element is stretchable under normal use conditions - e.g. when subjected to pressures below 2.5 atm. The stretchability of the balloon element is that it requires a strength of between about 0.1 MPa to about 10.0 MPa to extend the balloon to twice its length - preferably between about 0.2 MPa to about 1.5 MPa. In other words, when the strain has reached 100% of the initial length, then the tension in the material is between about 0.1 MPa and about 10.0 MPa - preferably between about 0.2 MPa and about 1.5 MPa.

In another embodiment the material is not stretchable in the normal use-condition that is when subjected to internal pressures below 2.5 atm. This means that the balloon element may be a moulded element made of a material which is not stretchable. The same kind of materials as mentioned above may be used for this embodiment with the addition that they need not be stretchable. Furthermore, materials like PET (polyethylene teraphthalate), PE (poly-ethylene), PP (polypropylene), Nylon, styrene and TPU may be used.

In these embodiments the pressure-sensitive valve will open at a pressure of about 2.5 atm. The valve will close again if the pressure inside the balloon drops below 1.5 atm.

The retention means (in the form of a lumen-defining element, e.g. a balloon) are attached to the shaft part below the outlet from the shaft water channel and the tip part above the inlet to the tip water channel. The retention means are preferably annually attached to the shaft part and the tip part respectively. The attachment should be liquid-tight in the normal use conditions e.g. when subjected to pressures below 2.5 atm.

The pressure-sensitive valve comprises one or more movable parts, which have their movements countered by resilient means.

If the tip part and the shaft part are separate parts, these resilient means bias the tip part and the shaft part together in the first configuration of the probe. The stress in the resilient means will act as a retaining force as the tip part is actuated in the longitudinal direction. Furthermore, as the water flow into the probe is stopped, the resilient means will bias the tip part to the first configuration thereby closing the access to the tip channel. In this embodiment, the user pumps irrigation liquid into the probe and the balloon element will expand or unfold predominantly in the transverse direction and subsequently in the longitudinal direction. The balloon element will expand or unfold until the pressure inside the balloon reaches a predetermined level - typically around 2.5 atm. At this point the movable part of the pressure-sensitive valve will be able to overcome the counterforce in the resilient means and urge the tip part from the shaft part thereby opening for the inlet to the tip water channel. Thus the tip water channel will be in fluid communication with the lumen of the balloon and irrigation fluid will be able to flow from the balloon lumen and through the tip water channel out through the eyelets and into the rectum or colon. Due to the retention means being attached to both the tip-part and the shaft part these means will keep the tip part from separating completely from the probe.

If the tip part and the shaft part are one integral element, the valve comprises a blocking element. During use the user will pump irrigation liquid into the probe and the balloon element will expand initially in the transverse direction until a predetermined pressure (e.g. approx. 2.5 atm.) is reached. When this pressure is reached, the pressure at the movable part(s) of the valve will be able to overcome the spring resistance (counterforce) of the resilient element and the blocking element will be displaced thus uncovering the inlet to the tip channel. The tip channel will then be in fluid communication with the lumen (or the 1tip channel will be opened) and the irrigation liquid will be able to flow through the tip water channel, out through the eyelets and further into the rectum or colon.

During use of the probe, the pressure will be equalized in the valve and a balance between the pressure of the resilient means and the pressure inside the balloon will be achieved and the inlet to the tip water channel will be maintained in an open position.

In an embodiment the resilient element is a spring. Other embodiments relates to the resilient element being a liquid-dampening element or material, which in itself is elastic. Any other resilient elements well known in the art may also be used.

The valve may further include an adjustment screw for adjusting the resilience in the resilient element. Thereby the user will be able to control the counterforce and thereby the pressure needed to overcome this counterforce. Such adjustment screws are well-known in the art.

The irrigating liquid may be any suitable medium such as tap water, isotonic salt water, sterile water or oily substances.

In another aspect the invention concerns a method of performing anal irrigation using a probe comprising a shaft part, a tip part and retention means, which are attached to the tip part and the shaft part and define a lumen there between, where the shaft part and the tip part are generally elongated elements defining a longitudinal direction, which method comprises the steps of
- introducing the probe into the rectum,
- supplying irrigation fluid into the probe through a water channel of the shaft part and into the lumen
where the irrigation fluid will create a pressure in the retention means and as this pressure reaches a predetermined level, the pressure will cause the probe to move between a first configuration, in which the inlet to the tip channel is not in fluid communication with the lumen, and a second configuration, in which the inlet to the tip channel is in fluid communication with the lumen.

This method is also easy to use, as the user only needs to pump water into the probe and then the rest (the expansion and the opening of the water channel) will happen automatically. Again the user may use the same pumping action for pumping the retention means as well as the irrigation fluid.

### Brief Description of the Drawings

Figures 1 a and 1 b illustrate an embodiment of the invention in which the tip part and shaft part are separate elements; and
Figures 2a and 2b illustrate an embodiment in which the tip part and the shaft part are one integral element;

### Detailed Description of the Drawings

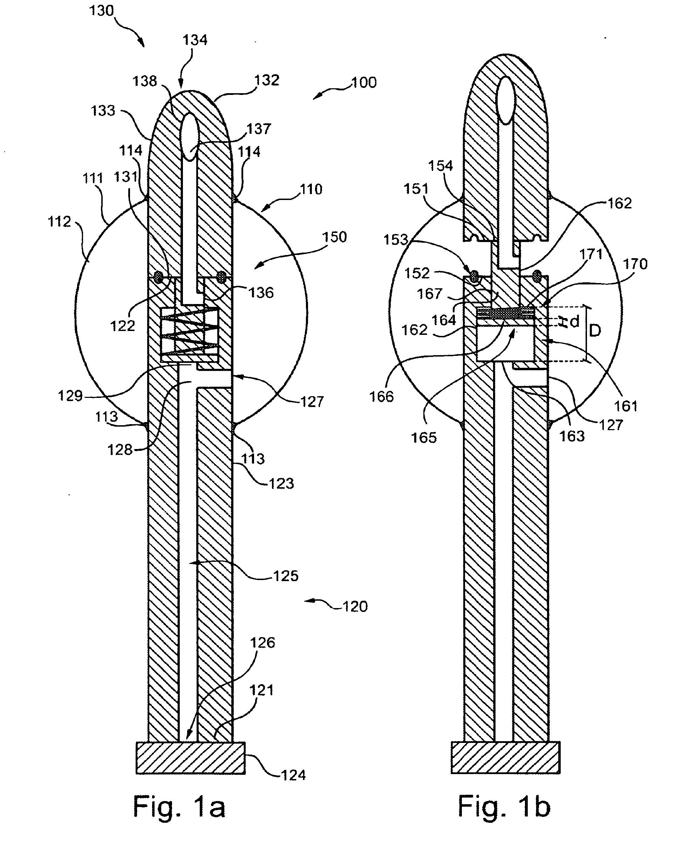

Figure 1 illustrates an embodiment of the invention in which the tip part and the shaft part are separate elements. Figure 1a illustrate the first configuration and figure 1b the second configuration. The probe 100 comprises a retention element 110 surrounding the joint **1**50 between the tip part 130 and the shaft part 120. The probe further comprises a pressure-sensitive valve 160, which in this embodiment is placed in the joint 150 between the shaft part 120 and the tip part 130 of the probe. The shaft part 120 is defined as the part connected to the water supply and the tip part is the other part. When defining the proximal end of the probe as the insertion end and the distal end as the other end, then the tip part 130 corresponds to the proximal part of the probe and the shaft part 120 corresponds to the distal part of the probe. The retention element 110 comprises in the illustrated embodiment a balloon 111 defining a balloon lumen 112 between the inside of the balloon, the tip part and the shaft part. The shaft part 120 has a distal end 121 and a proximal end 122 and a curved surface 123 extending therebetween such that the ends 121, 122 and the surface 123 combined makes out a generally cylindrical element. The shaft part 120 also comprises a connector 124 in the distal end and a water channel 125 with a water inlet 126 and a balloon outlet 127. In the embodiment illustrated in figure 1 the water channel 125 comprises two outlets, a balloon outlet 127 and a cavity outlet 129 into balloon lumen 112 and a cavity 161 of the valve. The tip part 130 comprises a distal end 131 and a proximal end 132 and a curved surface 133 extending there between. Like with the shaft part 120 these three parts make out a generally cylindrical element. The proximal end 132 corresponds to the actually tip 134 of the probe. The tip part 130 further comprises a water channel 135 having a balloon inlet 136 and a tip outlet 137. The tip outlet 137 is provided in connection with eyelets 138 at the tip 134 of the probe. The joining part 150 comprises a ring surface 151 at the distal end 131 of the tip part 130 and a corresponding ring surface 152 at the proximal end 121 of the shaft part 120. The two ring surfaces 151, 152 are closely attached in a first configuration of the probe, while they are out of touch in a second configuration of the probe. The joining part 150 further comprises a sealing 153 between the two ring surfaces 151, 152.

The valve comprises a cavity 161 into which the irrigation liquid flows. This cavity may be formed as a cylinder having a curved wall 162 or as a box-like cavity having side walls 162. The bottom of the cavity has an inlet 163 corresponding to the cavity outlet 129 of the shaft water channel 125. A part of a movable valve-element 164 extends inside the cavity 161, so that a pressure area 165 of the movable valve-element 164 faces the inlet 163. The pressure area 165 is in this embodiment constituted by the bottom surface of an abutment 166 situated on a cylindrical element 167, which is made integral with the tip part 130. Thereby pressure exerted on the pressure area 165 and at the abutment 166 will be transferred through the cylindrical element 167 to the tip part 130. This way the tip part 130 will be displaced longitudinally under influence of pressure exerted on the pressure area 165. The abutment 166 is movable within the cavity 161 as the length in the longitudinal direction D of the cavity exceeds the corresponding length d of the abutment - see figure 1 b. The movable valve-element 164 further comprises a resilient element 170 in this embodiment illustrated as a spring 171. This resilient element 170 acts as a counterforce to the pressure exerted on the pressure area 165 so that only when the pressure exceeds the resilience of the resilient element 170 the movable valve-element 164 will be able to be displaced.

The inlet 136 to the tip water channel 135 is placed in the cylindrical element 167. Thus to open to the inlet 136 the tip part 130 has to be displaced in the longitudinal direction with respect to the shaft part 120.

When the probe is to be used, the probe with the balloon in the collapsed position is inserted into the rectum. Water supply means are attached to the connector 124 at the distal end of the shaft part 120 of the probe 100. Initially the irrigation fluid will flow through the water channel 125 and through the balloon outlet 127 into the balloon and also through the cavity outlet 129 into the cavity 161. When the pressure in the probe has reached a predetermined level then the pressure exerted on the pressure area 165 will be able to overcome the spring force of the resilient element 170 and thus the abutment 166 will be displaced longitudinally. Thereby the cylindrical element 167 connecting the abutment and the tip part 130 will also be displaced thus eventually opening the inlet 136 to the tip water channel. Then water will flow through the balloon lumen 112 and through the inlet 136 to the tip water channel, through the tip water channel 135 and out through the eyelets 138. The tip part 130 is kept in contact with the shaft part 120 through the abutment 166 in the cavity 161 and through the retention means 111.

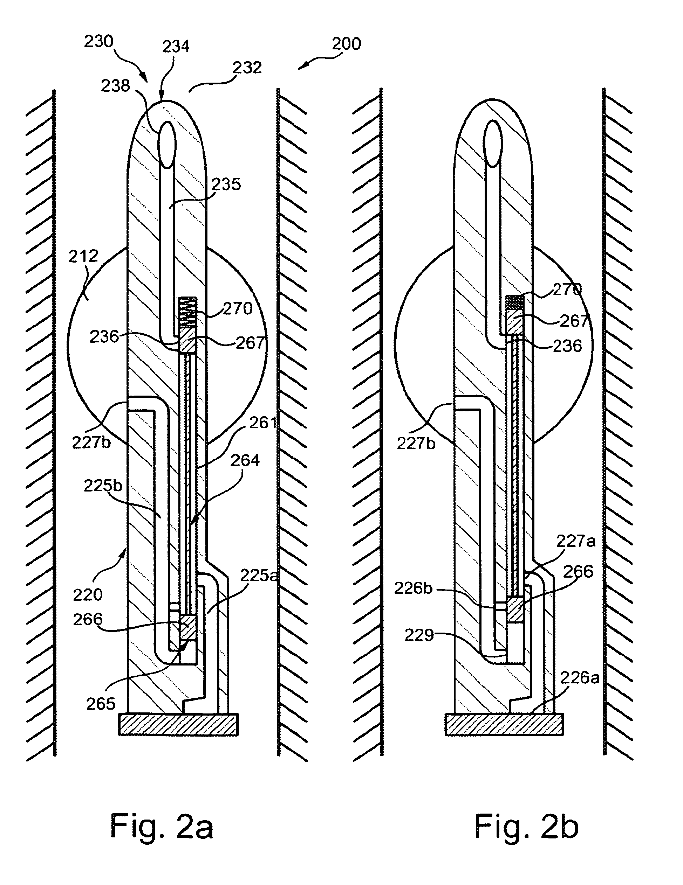

Figure 2 illustrates a probe 200 according to the invention in which the tip part 230 and the shaft part 220 are one integral element. In this figure the same reference numbers as in figure 1 are used for corresponding parts except for the prefix 2. The two parts 220, 230 comprise separate water channels so that the tip part 230 has a tip water channel 235 and the shaft part 220 has a first shaft water channel 225a which is in communication with the connector and a second shaft water channel 225b which is in communication with the balloon lumen 212. In the first configuration of the probe the two shaft water channels are in communication with each other- see figure 2a. In the second configuration of the probe, the first shaft water channel 225a is in communication with the tip water channel 235 but not in communication with the second shaft water channel 225b - see figure 2b.

The probe further comprises a valve 260, which in this embodiment has a movable valve element 264 situated in a cavity 261. In this embodiment the cavity consists of a channel having sidewalls 262. The cavity 261 has an inlet 263 corresponding to a cavity outlet 229 from the second shaft water channel 225b. The movable valve element 264 further comprises two blocking elements, a tip blocking element 267 and a shaft blocking element 266. The two blocking elements, 266, 267 are connected by a rod element 268, which is non-resilient under the normal use conditions. In this embodiment, the bottom surface of the shaft blocking element 266 defines the pressure area 265 on which pressure is exerted to get the movable valve element 264 to be displaced. The tip blocking element 267 is in this embodiment connected to a resilient element 270 at the side opposite the rod connection. As mentioned in the description of figures 1 a and 1 b this resilient element will act as a counterforce to the pressure exerted on the tip blocking element 267 through the rod element 268 and the shaft blocking element 266.

The first shaft water channel 225a has an inlet 226a in the connector 224 and an outlet 227a in communication with a channel defining the cavity 261 of the valve 260. The second shaft water channel 225b has an inlet 226b in communication with the channel defining the cavity 261 and two outlets - one in communication with the balloon lumen 212, a balloon outlet, 227b and another in communication with cavity 261 and more or less facing the pressure area 265, a cavity outlet 229.

When the probe is to be used, irrigation liquid is pumped into the first shaft water channel 225a through the inlet 226a and out through the outlet 227a. From there irrigation liquid will flow into the cavity 261 and through the inlet 226b to the second shaft water channel 225b. It will then flow into the balloon lumen 212 through the balloon outlet 227b. When pressure builds up inside the probe 200 as the balloon fills, the irrigation liquid will exert a larger and larger pressure on the pressure area 265 through the cavity outlet 229. At some point this pressure will exceed the counterforce of the resilient element 270 and the movable valve element 264 will be displaced towards the tip part 230. Thereby the tip blocking element 267 will be removed from the inlet 236 to the tip water channel and the shaft blocking element 266 will block the inlet 226b to the second shaft water channel 225b thereby to a certain degree maintaining the pressure inside the balloon 212 and at the pressure area 265.Thus irrigation liquid will flow from the first shaft water channel 225a through the cavity 261 and through the tip water channel 235 and further through the eyelets 238 into the rectum or stoma. At some point the pressure exerted on the pressure area 265 may be reduced - due to movement of the probe of the balloon - and thus the counterforce in the resilient element will force the blocking elements back to the first configuration of the probe. Continuous pumping will again build up the pressure at the pressure area 265 eventually causing the movable valve-element 264 to move in direction of the tip part and thus opening for the inlet 236 to the tip water channel. This process may repeat itself several times during the irrigation procedure but it has no influence on the treatment. As long as the user continues to pump, eventually enough irrigation liquid will have flowed into the rectum or stoma.

## Claims

1. An anal probe (100, 200) for performing anal irrigation having a tip part (130, 230) and a shaft part (120, 220), which probe (100, 200) comprises
- a tip part (130, 230) having a tip channel (135, 235) with an inlet (136, 236) and an outlet (137, 237)
- a shaft part (120, 220) having a shaft channel (125, 225a, 225b) with an inlet (126, 226a, 226b), an outlet (127, 227b) and a connector (124, 224) adapted for connection to fluid supply means
- retention means (110, 210) for retaining the probe (100, 200) in the rectum, which retention means (110, 210) is attached to the tip part (130, 230) and the shaft part (120, 220) and lumen is defined by the retention means (110, 210), the tip part (130, 230) and the shaft part (120, 220), where the outlet (127, 227b) of the shaft is in fluid communication with the lumen (112, 212) of the retention means and **characterized in that** the probe (100, 200) comprises a pressure-sensitive valve (160, 260) adapted for providing the probe with
- a first configuration in which the inlet (136, 236) to the tip channel is not in fluid communication with the lumen (112, 212), and
- a second configuration in which the inlet (136, 236) to the tip channel is in fluid communication with the lumen (112, 212).

2. An anal probe (100, 200) according to claim 1 wherein the pressure-sensitive valve (160, 260) comprises one or more movable parts (164, 264), which have their movement countered by resilient means (170, 270).

3. An anal probe (100, 200) according to claims 1 or 2 where the tip part (130, 230) and the shaft part (120, 220) are separate parts.

4. An anal probe (100, 200) according to claim 3 where tip part (130, 230) and the shaft part (120, 220) has contact surfaces (151, 152) in contact with each other in the first configuration of the probe.

5. An anal probe (100, 200) according to any of claims 3 or 4 where the tip part (130, 230) and the shaft part (120, 220) are elongated elements defining a longitudinal direction and where the tip part (130, 230) is displaced longitudinally with respect to the shaft part (120, 220) in the second configuration of the probe.

6. An anal probe (100, 200) according to any of claims 3-5 where the inlet (136) to the tip part is placed in the contact surface (151) of the tip part.

7. An anal probe (100, 200) according to any of claims 3-6 where either the tip part (130, 230) or the shaft part (120, 220) has a protrusion and the other has an indentation corresponding to the protrusion.

8. An anal probe (100, 200) according to claim 7 where the protrusion comprises an abutment (166) and the indentation comprises a cavity (161).

9. An anal probe (100, 200) according to any of the preceding claims where the retention means (110, 210) comprises a balloon (111, 211).

10. An anal probe (100, 200) according to claim 9 where the balloon (111, 211) is made of a stretchable material.

11. An anal probe (100, 200) according to claim 1 or 2 where the tip part (130, 230) and the shaft part (120, 220) are one integral part.

12. An anal probe (100, 200) according to claim 11 wherein the movable parts (164, 264) of the pressure-sensitive valve include a blocking element (267) which in the first configuration of the probe covers the inlet (236) to the tip water channel and in the second configuration of the probe is displaced with respect to the inlet.

## Patentansprüche

1. Analsonde (100, 200) zur Durchführung einer Analspülung mit einem Spitzenteil (130, 230) und einem Schaftteil (120, 220), wobei die Sonde (100, 200) Folgendes umfasst:
- einen Spitzenteil (130, 230) mit einem Spitzenkanal (135, 235) mit einem Einlass (136, 236) und einem Auslass (137, 237)
- einen Schaftteil (120, 220) mit einem Schaftkanal (125, 225a, 225b) mit einem Einlass (126, 226a, 226b), einem Auslass (127, 227b) und einem Verbindungsglied (124, 224) zur Verbindung mit einem Flüssigkeitszuführungsmittel
- Haltemittel (110, 210) zum Halten der Sonde (100, 200) im Rektum, wobei das Haltemittel (110, 210) am Spitzenteil (130, 230) und am Schaftteil (120, 220) befestigt ist und ein Lumen vom Haltemittel (110, 210), dem Spitzenteil (130, 230) und dem Schaftteil (120, 220) definiert wird,
worin der Auslass (127, 227b) des Schaftes mit dem Lumen (112, 212) des Haltemittels in Flüssigkeitsverbindung steht und **dadurch gekennzeichnet, dass** die Sonde (100, 200) ein druckempfindliches Ventil (160, 260) umfasst, das so ausgelegt ist, dass es die Sonde in
- eine erste Konfiguration, in welcher der Einlass (136, 236) zum Spitzenkanal nicht mit dem Lumen (112, 212) in Flüssigkeitsverbindung steht, und in
- eine zweite Konfiguration bringen kann, in welcher der Einlass (136, 236) zum Spitzenkanal mit dem Lumen (112, 212) in Flüssigkeitsverbindung steht.

2. Analsonde (100, 200) nach Anspruch 1, worin das druckempfindliche Ventil (160, 260) ein oder mehr bewegliche Teile (164, 264) umfasst, deren Bewegung von nachgiebigen Mitteln (170, 270) gekontert wird.

3. Analsonde (100, 200) nach Anspruch 1 oder 2, worin der Spitzenteil (130, 230) und der Schaftteil (120, 220) getrennte Teile sind.

4. Analsonde (100, 200) nach Anspruch 3, worin der Spitzenteil (130, 230) und der Schaftteil (120, 220) Kontaktflächen (151, 152) aufweisen, die in der ersten Konfiguration der Sonde miteinander in Berührung sind.

5. Analsonde (100, 200) nach einem der Ansprüche 3 oder 4, worin der Spitzenteil (130, 230) und der Schaftteil (120, 220) längliche Elemente sind, die eine Längsrichtung definieren, und worin der Spitzenteil (130, 230) in der zweiten Konfiguration der Sonde relativ zum Schaftteil (120, 220) in Längsrichtung verschoben wird.

6. Analsonde (100, 200) nach einem der Ansprüche 3-5, worin der Einlass (136) zum Spitzenteil in der Kontaktfläche (151) des Spitzenteils angeordnet ist.

7. Analsonde (100, 200) nach einem der Ansprüche 3-6, worin entweder der Spitzenteil (130, 230) oder der Schaftteil (120, 220) einen Vorsprung aufweist und der jeweils andere eine dem Vorsprung entsprechende Vertiefung aufweist.

8. Analsonde (100, 200) nach Anspruch 7, worin der Vorsprung ein Widerlager (166) und die Vertiefung einen Hohlraum (161) aufweist.

9. Analsonde (100, 200) nach einem der vorhergehenden Ansprüche, worin das Haltemittel (110, 210) einen Ballon (111, 211) umfasst.

10. Analsonde (100, 200) nach Anspruch 9, worin der Ballon (111, 211) aus einem dehnbaren Material besteht.

11. Analsonde (100, 200) nach Anspruch 1 oder 2, worin der Spitzenteil (130, 230) und der Schaftteil (120, 220) ein einstückiges Teil sind.

12. Analsonde (100, 200) nach Anspruch 11, worin die beweglichen Teile (164, 264) des druckempfindlichen Ventils ein Blockierelement (267) aufweisen, das in der ersten Konfiguration der Sonde den Einlass (236) zum Spitzenwasserkanal abdeckt und in der zweiten Konfiguration der Sonde relativ zum Einlass verschoben wird.

## Revendications

1. Sonde anale (100, 200) pour réaliser une irrigation anale, comprenant une partie de tête (130, 230) et une partie de tige (120, 220), ladite sonde (100, 200) comprenant:
- une partie de tête (130, 230) présentant un canal de tête (135, 235) comportant une entrée (136, 236) et une sortie (137, 237);
- une partie de tige (120, 220) présentant un canal de tige (125, 225a, 225b) comportant une entrée (126, 226a, 226b), une sortie (127, 227b) et un connecteur (124, 224) adapté pour être connecté à des moyens d'alimentation en fluide;
- des moyens de retenue (110, 210) pour retenir la sonde (100, 200) dans le rectum, lesdits moyens de retenue (110, 210) étant attachés à la partie de tête (130, 230) et à la partie de tige (120, 220), et une lumière est définie par les moyens de retenue (110, 210), la partie de tête (130, 230) et la partie de tige (120, 220),
dans laquelle la sortie (127, 227b) de la tige est en communication fluidique avec la lumière (112, 212) des moyens de retenue, et **caractérisée en ce que** la sonde (100, 200) comprend une valve sensible à la pression (160, 260) qui est adaptée pour placer la sonde dans:
- une première configuration, dans laquelle l'entrée (136, 236) du canal de tête n'est pas en communication fluidique avec la lumière (112, 212), et
- une deuxième configuration, dans laquelle l'entrée (136, 236) du canal de tête est en communication fluidique avec la lumière (112, 212).

2. Sonde anale (100, 200) selon la revendication 1, dans laquelle la valve sensible à la pression (160, 260) comprend une ou plusieurs partie(s) mobile(s) (164, 264) dont le déplacement est contrecarré par des moyens élastiques (170, 270).

3. Sonde anale (100, 200) selon la revendication 1 ou 2, dans laquelle la partie de tête (130, 230) et la partie de tige (120, 220) sont des parties séparées.

4. Sonde anale (100, 200) selon la revendication 3, dans laquelle la partie de tête (130, 230) et la partie de tige (120, 220) présentent des surfaces de contact (151, 152) qui sont en contact l'une avec l'autre dans la première configuration de la sonde.

5. Sonde anale (100, 200) selon l'une quelconque des revendications 3 ou 4, dans laquelle la partie de tête (130, 230) et la partie de tige (120, 220) sont des éléments allongés qui définissent une direction longitudinale, et dans laquelle la partie de tête (130, 230) est déplacée de façon longitudinale par rapport à la partie de tige (120, 220) dans la deuxième configuration de la sonde.

6. Sonde anale (100, 200) selon l'une quelconque des revendications 3 à 5, dans laquelle l'entrée (136) de la partie de tête est placée dans la surface de contact (151) de la partie de tête.

7. Sonde anale (100, 200) selon l'une quelconque des revendications 3 à 6, dans laquelle soit la partie de tête (130, 230), soit la partie de tige (120, 220) comporte une saillie, et l'autre présente une indentation qui correspond à la saillie.

8. Sonde anale (100, 200) selon la revendication 7, dans laquelle la saillie comprend une butée (166), et l'indentation comprend une cavité (161).

9. Sonde anale (100, 200) selon l'une quelconque des revendications précédentes, dans laquelle les moyens de retenue (110, 210) comprennent un ballonnet (111, 211).

10. Sonde anale (100, 200) selon la revendication 9, dans laquelle le ballonnet (111, 211) est constitué d'un matériau étirable.

11. Sonde anale (100, 200) selon la revendication 1 ou 2, dans laquelle la partie de tête (130, 230) et la partie de tige (120, 220) forment une partie intégrale.

12. Sonde anale (100, 200) selon la revendication 11, dans laquelle les parties mobiles (164, 264) de la valve sensible à la pression comprennent un élément de blocage (267) qui, dans la première configuration de la sonde, couvre l'entrée (236) jusqu'au canal d'eau de tête et, dans la deuxième configuration de la sonde, est déplacé par rapport à l'entrée.
